# EUROPÄISCHE PATENTSCHRIFT

(11) **EP 3 015 063 B1**
(45) Veröffentlichungstag und Bekanntmachung des Hinweises auf die Patenterteilung: **14.06.2023**
(21) Anmeldenummer: 14003687.2
(22) Anmeldetag: 31.10.2014
(51) Int. Cl.: A61B 5/294, A61N 1/04, A61B 5/262

(54) **Vorrichtung zur Diagnose der Funktionalität von Nervenfasern**
Device for the diagnosis of the functionality of nerve fibres
Dispositif de diagnostic de la fonctionnalité des fibres nerveuses

(43) Veröffentlichungstag der Anmeldung: 04.05.2016
(73) Patentinhaber: Rheinisch-Westfälische Technische Hochschule (RWTH) Aachen, 52062 Aachen (DE)
(72) Erfinder: Rolke, Roman, 53105 Bonn (DE); Mücke, Martin, 40724 Hilden (DE)
(74) Vertreter: König Szynka Tilmann von Renesse Patentanwälte Partnerschaft mbB Düsseldorf

(56) Entgegenhaltungen:
- US-A1- 2006 276 720
- US-A1- 2007 185 409
- US-A1- 2007 208 268
- US-A1- 2008 051 673
- US-A1- 2010 094 378
- US-A1- 2012 101 358
- US-A1- 2012 226 330

## Beschreibung

Die Erfindung betrifft eine Vorrichtung zur Diagnose der Funktionalität von Nervenfasern, insbesondere kann mit der Vorrichtung ein somatosensibel evoziertes Potential (SEP) dünner Nervenfasern (Small-fibers, Aδ- und C-Fasern) ermittelt werden.

Mit derartigen Vorrichtungen kann der funktionelle Zustand von Nervenfasern (lokal) diagnostiziert werden. Es kann diagnostiziert werden, ob eine Schädigung und/oder Fehlfunktion dünner Nervenfasern vorliegt. Insbesondere kann unter Verwendung einer derartigen Vorrichtung ein SEP für dünne Nervenfasern (Small-fibers) ermittelt werden.

Bei somatosensibel evozierten Potentialen (SEP) handelt es sich um eine elektrische Antwort von mittels insbesondere einer elektrischen Reizung angeregten Nervenfasern im Verlauf der Schleifenbahn. Die elektrische Antwort wird zumeist in Höhe des Eintritts in das Rückenmark und über dem betreffenden Hirnareal abgeleitet.

Es ist bekannt, für die elektrische Reizung eine Reizelektrodenanordnung zu verwenden, die zwei zueinander konzentrische Ringe als Anode und Kathode aufweist. Die bekannte Reizelektrodenanordnung wird von einer Einheit mit elektrischen Pulsen beaufschlagt und die elektrischen abgeleiteten Signale werden von einer weiteren Einheit erfasst. Um einen Verlauf eines Small-fiber SEP zu erhalten, sind derzeit mehrere Messungen mit einer jeweiligen Messaufnahme des Signals nötig, wobei die einzelnen Messungen addiert und mathematisch vor einer Ausarbeitung aufbereitet werden müssen, welches aufwändig ist und mit Fehlern behaftet sein kann. Daher hat sich die Verwendung der bekannten Reizelektrodenanordnung als schwierig erwiesen, da das mathematisch bearbeitete Signal nicht eindeutig als reines Small-fiber SEP identifizierbar ist. Durch die mathematische Prozessierung und Aufbereitung der aufgenommenen Messwerte können Artefakte und/oder die Art der mathematischen Aufbereitung zu einer Verfälschung des über mehrere Messungen "gemittelten" Spannungsverlaufs führen, so dass die im Ergebnis erhaltene Kurve eventuell nicht das eigentliche Small-fiber SEP wiedergibt.

Ein Small-fiber SEP ist für eine Diagnose deswegen interessant, weil ein Standard-SEP nur die Funktion z.B. dicker Aβ-Fasern anzeigt, die nicht mit einer Schmerzwahrnehmung korreliert sind. Aβ-Fasern vermitteln Berührung und Vibration. Mit einem Small-fiber SEP ist eine objektive Darstellung einer Faserfunktion im Schmerzsystem möglich.

Vorrichtungen zur Diagnose der Funktionalität von Nervenfasern entsprechend des Stands der Technik werden zum Beispiel in US2007/0185409A1, US2008/0051673A1, US2012/0101358A1, US2007/0208268A1, US2012/0226330A1, US2010/0094378A1 und US2006/0276720A1 beschrieben.

Vor diesem Hintergrund liegt der Erfindung die Aufgabe zugrunde, eine verbesserte Vorrichtung zur Diagnose der Funktionalität von Nervenfasern bereitzustellen, die im Hinblick auf die Qualität des gemessenen Signals verbessert ist, insbesondere eine Aufnahme eines Small-Fiber SEP in real-time (Echtzeit) ermöglicht.

Diese Aufgabe wird durch den Gegenstand des unabhängigen Anspruchs gelöst. Vorteilhafte Ausführungsformen sind in den Unteransprüchen und der hiernach folgenden Beschreibung wiedergegeben.

Die Vorrichtung zur Diagnose der Funktionalität von Nervenfasern weist eine Elektrodenanordnung zur Reizbeaufschlagung, die mehrere in einem Elektrodenfeld angeordnete Kathoden und mindestens eine zu den Kathoden beabstandete Anode umfasst, und mindestens eine Ableitelektrode auf.

Mit der genannten Ausgestaltung der Elektrodenanordnung zur Reizbeaufschlagung ist es erstmals möglich, ein real-time Small-fiber SEP zu messen, also ein SEP, mit dem die Funktionalität von dünnen Nervenfasern (Aδ- und C-Fasern) quantitativ bewertet werden kann. Die Vorrichtung kann für eine objektive Darstellung einer Faserfunktion im Schmerzsystem verwendet werden. Eine, der eigentlichen Messung nachfolgende, aufwändige mathematische Verarbeitung von zu unterschiedlichen Zeitpunkten aufgenommener Messsignale zur Erzeugung eines Potentialsignals - wie es aus dem Stand der Technik bekannt ist - kann entfallen. Durch die nunmehr nicht mehr notwendige mathematische Ver- und Bearbeitung ist es möglich, das Small-fiber SEP an sich zu erfassen ohne eventuell mittels mathematischer Bearbeitung Veränderungen hieran vorzunehmen.

Ferner ermöglicht die genannte Ausgestaltung der Elektrodenanordnung zur Reizbeaufschlagung die Verwendung eines Standard-"SEP-Geräts", an dem die Elektrodenanordnung zur Reizbeaufschlagung und die eine oder mehreren Ableitelektroden angeschlossen werden können. Das Standard-"SEP-Gerät" dient der elektrischen Beaufschlagung der Elektrodenanordnung zur Reizbeaufschlagung und dem Erfassen des Signals der Ableitelektrode(n). Durch die Verwendung eines Geräts liegt eine implizite Kopplung zwischen Reizbeaufschlagung und abgeleitetem Potential vor. Das Gerät liefert inhärent den "Triggerimpuls", wenn die Reizbeaufschlagung gestartet wird und es kann intern festgestellt werden, wieviel Zeit von der Reizbeaufschlagung bis zum Erhalt des Small-fiber SEP bzw. einzelner Bestandteile des Small-fiber SEP (vorbestimmte Peaks des SEP) vergeht.

Mit der erfindungsgemäßen Vorrichtung kann die Antwort von dünnen Nervenfasern ("small fiber") auf eine elektrische Reizung mit der genannten Elektrodenanordnung zur Reizbeaufschlagung erfasst werden. Dies ist vor allem deshalb interessant, weil es u.a. im Rahmen einer Chemotherapie bei Tumorpatienten zu Schmerzen oder Missempfindungen kommt, die durch Schädigung dünner Nervenfasern entstehen. Konventionelle neurologische Diagnostika, z.B. in Form der Neurographie oder Quantitativ sensorischen Testung (QST) entgehen diese Schädigungen, weil diese Verfahren überwiegend die Funktion dick myellinisierter Fasern abbilden. Diese Problematik besteht nicht nur bei Tumorpatienten, sondern bei allen Schädigungen dünner Nervenfasern, wie beispielsweise einer Polyneuropathie mit Schädigung dünner Nervenfasern (z.B. Diabetischer Polyneuropathie, HIV-Neuropathie, Guillain-Barré-Syndrom, Churg-Strauss-Syndrom).

Sofern sich in den Patentansprüchen oder der Beschreibung der Begriff "mindestens ein" in entsprechenden grammatikalischen Ausprägungen im Hinblick auf das in Bezug genommene Nomen findet, so umfasst dieser Begriff genau eines oder mehrere, d.h. zwei, drei usw., der durch das Nomen bezeichneten Elemente.

Vorzugsweise bilden die in einem Elektrodenfeld angeordneten Elektroden die Kathoden und die weitere Elektrode bildet die Anode. Im Sinne der Erfindung ist unter einer Kathode bzw. einer Anode eine mit entsprechender Polarität beaufschlagte Elektrode zu verstehen. Vorzugsweise wechselt eine Elektrode das Vorzeichen bei einer Strom- bzw. Spannungsbeaufschlagung nicht. Es kann aber auch vorgesehen sein, dass eine Elektrode mit Strom-/Spannungspulsen beaufschlagt wird, deren Polarität sich ändert.

Die Elektrodenanordnung zur Reizbeaufschlagung kann auf die Haut im zu untersuchenden Bereich (Körperteil, Körperbereich, beispielsweise in Form von Arm, Hand, Finger) aufgelegt bzw. in Richtung der Haut gedrückt werden. Die Elektrodenanordnung ist derart ausgestaltet, dass die Haut nicht perforiert bzw. auch nicht teilweise durchdrungen wird. Die Elektroden des Elektrodenfelds weisen eine verjüngende Oberfläche auf. Die Oberfläche kann kugelförmig, halbkugelförmig oder kegelförmig zulaufend zur Anlage auf der Haut ausgebildet sein, wodurch die Emission des elektrischen Feldes in die Haut verbessert werden kann. Elektroden des Elektrodenfelds können als sogenannte Ball-Grids ausgestaltet sein. Die Elektroden des Elektrodenfelds können zwischen spitz, stumpf, abgerundet und eckig variiert werden. Es sind aber auch andere Formen für die Elektroden des Elektrodenfelds möglich.

Die Elektroden des Elektrodenfelds können auf einer, insbesondere biegsamen, Platine, beispielsweise als Ball-Grids, ausgebildet sein, welches die Herstellung der Elektrodenanordnung zur Reizbeaufschlagung vereinfacht.

Die Elektroden des Elektrodenfelds sind mit der verjüngenden Oberfläche für eine punktuelle Kontaktierung der Haut ausgestaltet. Eine Elektrode kann als Kontaktpunkt zur Anlage auf der Haut ausgestaltet sein. Die einzelnen Kontaktpunkte, die Elektroden, weisen jeweils einen Durchmesser von bis zu 1,5 mm auf. Die einzelnen Kontaktpunkte können jeweils in einem Abstand von wenigen Millimetern bis Zentimetern, insbesondere zwischen 2,0 mm bis 10 mm, besonders bevorzugt 3,0 mm bis 7,5 mm, vorzugsweise etwa 4,5 mm, angeordnet sein. Das Elektrodenfeld kann in Spalten oder in Zeilen angeordnete Elektroden aufweisen. Es kann aber auch vorgesehen sein, dass die Elektroden des Elektrodenfelds ohne erkennbar gleichmäßiges Muster flächig verteilt sind. Erfindungsgemäß wird von dem Begriff Elektrodenfeld eine flächige Verteilung von punktuell ausgebildeten Elektroden erfasst. Vorzugsweise überdeckt das Elektrodenfeld einen Bereich von 1 cm² bis 25 cm², vorzugsweise etwa 2 cm² bis 4 cm². Vorzugsweise können auf dem Feld bzw. der Fläche 2 bis 100 Elektroden, insbesondere 10 bis 50 Elektroden, ausgebildet sein. Eine Elektrodendichte des Elektrodenfelds von mindestens 2/cm², bevorzugt mindestens 3/cm², und insbesondere bevorzugt mindestens 4/cm² kann erfindungsgemäß vorgesehen sein. Es gab gute Ergebnisse bei etwa 3 bis 5 Elektroden pro 1 cm².

Das Elektrodenfeld kann eine äußere Kontur aufweisen, die von den äußeren Elektroden des Elektrodenfelds gebildet wird, und quadratisch ist, allerdings sind auch rechteckige Konturen bzw. Flächen, kurvige oder mittels Geradenzügen gebildete Konturen erfindungsgemäß umfasst. Insbesondere bevorzugt ist, dass die flächenförmige Anordnung der Elektroden flexibel hinsichtlich der Oberfläche der Elektroden, die in Anlage mit der Oberfläche des menschlichen oder tierischen Körpers gelangt, gestaltet ist, wodurch die Elektrodenanordnung auf die Oberfläche eines menschlichen Körpers ohne Kontaktverlust aufgebracht werden kann.

Es kann vorgesehen sein, dass die Elektrodenanordnung ein leitfähiges Textil oder eine leitfähige flexible Struktur, das in einem nicht leitfähigen Textil bzw. einem anderen Material eingebettet oder auf diesem aufgebracht ist, umfasst. Es kann vorgesehen sein, dass die Elektrodenanordnung leitfähige Fäden umfasst, die in einem nicht leitfähigen Textil oder anderem Material eingewirkt sind. Die Elektroden des Elektrodenfelds können als Abschnitte des leitfähigen Fadens ausgebildet sein. Hierdurch wird eine einfach handhabbare Elektrodenanordnung geschaffen, die einfach herstellbar ist.

Die zum Elektrodenfeld beabstandete weitere Elektrode wird als Gegenelektrode (Elektrode anderer Polarität als die Polarität der Elektroden des Elektrodenfelds) verwendet. Die weitere Elektrode ist vorzugsweise für eine flächige Anlage auf der Haut ausgestaltet. Es kann eine flächige Kontaktierung als Flächenelektrode oder Gelelektrode erfolgen; die weitere Elektrode kann als Flächenelektrode oder Gelelektrode ausgestaltet sein. Die Fläche der weiteren Elektrode als Gegenelektrode für das Elektrodenfeld kann insbesondere im Bereich des zweifachen der Fläche des Elektrodenfelds bis zum dreifachen der Fläche des Elektrodenfelds liegen. Es können auch noch größere Flächen für die weitere Elektrode gewählt werden.

Die weitere Elektrode kann vorzugsweise einen gegenüber einer Fläche ausgebildeten Vorsprung aufweisen, wodurch ermöglicht werden kann, dass mit dem Andrücken der weiteren Elektrode auf die Haut eine "Senke" in der Haut geschaffen wird, die eventuell auf der Haut vorhandene Flüssigkeit, insbesondere in Form von Schweiß, in der Senke im Bereich der weiteren Elektrode lokalisiert. Durch das Eindrücken der Haut im Bereich der weiteren Elektrode kann die Flüssigkeit dort "gesammelt" werden und die Gefahr eines Kurzschlusses zwischen Elektroden des Elektrodenfelds und der weiteren Elektrode wird verringert. Dieses gilt umso mehr, wenn der Abstand zwischen den äußeren Elektroden des Elektrodenfeld und der weiteren Elektrode gering ist.

Die Ableitelektrode kann als Nadelelektrode ausgestaltet sein. Es können zwei weitere Nadelelektroden als Ableitelektroden bzw. Referenzelektroden vorhanden sein, die beispielsweise im Bereich der Kopfhaut eingestochen werden. Es können aber auch Clip-Elektroden, insbesondere als Referenzelektroden zur Ableitelektrode verwendet werden, die den Vorteil aufweisen, dass weniger Nadelelektroden gestochen werden müssen.

Vorzugsweise sind die Elektroden des Elektrodenfelds in Zeilen und Spalten angeordnet, und die Anzahl der Zeilen ist im Bereich von 5 bis 13, insbesondere im Bereich 7 bis 11, und ganz besonders bevorzugt gleich 9, und die Anzahl der Spalten ist im Bereich von 1 bis 10, insbesondere im Bereich von 3 bis 7, ganz besonders bevorzugt gleich 5, wobei der Abstand der Zeilen im Bereich von wenigen Millimetern bis Zentimetern, insbesondere zwischen 2,0 mm bis 10 mm, besonders bevorzugt 3,0 mm bis 7,5 mm, vorzugsweise etwa 4,5 mm und der Abstand der Spalten im Bereich von wenigen Millimetern bis Zentimetern, insbesondere zwischen 2,0 mm bis 10 mm, besonders bevorzugt 3,0 mm bis 7,5 mm, vorzugsweise etwa 4,5 mm liegt. Der Abstand der Zeilen kann im Wesentlichen gleich dem Abstand der Spalten sein.

In einer bevorzugten Ausführungsform umgibt die weitere Elektrode die Elektroden des Elektrodenfelds zumindest teilweise, so dass ein von einem Schwerpunkt des Elektrodenfelds ausgehender virtueller Strahl über einen Winkelbereich von größer als 120 ° auf die weitere Elektrode auftreffen würde.

In einer bevorzugten Ausführungsform umgibt die weitere Elektrode die Elektroden des Elektrodenfelds geschlossen. Die weitere Elektrode kann in Form eines Linienzugs gebildet sein, der die Elektroden des Elektrodenfelds einschließt.

Vorzugsweise liegt der Abstand zwischen Elektroden des Elektrodenfelds, die zu der weiteren Elektrode benachbart sind, und der weiteren Elektrode im Bereich von 0,5 mm bis 6,0 mm, bevorzugt 0,5 mm bis 4,5 mm, insbesondere bevorzugt 0,7 mm bis 3,0 mm.

Vorzugsweise sind die Elektroden des Elektrodenfelds gleichmäßig im Elektrodenfeld angeordnet, wodurch eine gleichmäßige Reizung über den überdeckten Bereich erfolgen kann. Der Begriff "gleichmäßig" angeordnet umfasst eine im Wesentlichen homogene Dichte der Elektroden im Elektrodenfeld und/oder eine im Wesentlichen achssymmetrische und/oder punktsymmetrische Anordnung der Elektroden im Elektrodenfeld.

Vorzugsweise bildet eine kürzeste Verbindungslinie entlang benachbarter äußerer Elektroden des Elektrodenfelds eine symmetrische Fläche, wodurch eine homogene Reizung erzielt werden kann.

Bevorzugt variiert der Abstand zwischen den äußeren Elektroden des Elektrodenfelds und der weiteren Elektrode für die äußeren Elektroden. Die Kontur des Elektrodenfelds mit den äußeren Elektroden entspricht damit nicht der geometrischen Ausgestaltung der weiteren Elektrode in der Fläche.

Bevorzugt ist der Abstand zwischen den äußeren Elektroden des Elektrodenfelds und der weiteren Elektrode für die äußeren Elektroden im Wesentlichen gleich. Die Kontur des Elektrodenfelds mit den äußeren Elektroden entspricht damit der geometrischen Ausgestaltung der weiteren Elektrode in der Fläche.

Erfindungsgemäß sind weitere Elektroden identischer Polarität wie die der Elektroden des Elektrodenfeldes nicht ausgeschlossen und können vorhanden sein. Es kann auch vorgesehen sein, dass mehrere weitere Elektroden unterschiedlicher Polarität zu den Elektroden des Elektrodenfelds vorhanden sind.

Mit einer Anwendung der vorgenannten Vorrichtung ist es möglich, ein somatosensibles evoziertes Potential insbesondere für dünne Nervenfasern (Small-fiber) in Echtzeit zu messen.

Der Abstand zwischen den Elektroden des Elektrodenfelds und der weiteren Elektrode und/oder die Ausrichtung und/oder Lage zueinander kann mittels eines Haltemittels festgelegt sein, an dem die Elektroden des Elektrodenfeld und die weitere Elektrode angeordnet sind. Das Haltemittel kann beispielsweise eine flexible Fläche, eine Bandage, ein Band oder ein sonstiges Textil oder Gewirke sein.

Die Positionen der Elektroden des Elektrodenfelds können so gewählt werden, dass diese der Form des Körpers, an der das Elektrodenfeld in Anlage gelangen soll, entspricht. Es ist möglich, dass die Verteilung der Elektroden des Elektrodenfelds der Form eines zur Ausübung eines Druckes vorgesehenen Druckkörpers folgt. Beispielsweise können für unterschiedliche Stellen am Körper unterschiedliche Druckkörper vorgesehen sein.

Ein Druckkörper kann als Pelotte oder als Abstandsgewirke ausgestaltet sein. Eine Pelotte kann als Stütze für den Bereich der Anlage auf der Haut, insbesondere für ein Gelenk, dienen. Eine Pelotte kann einen elastischen Werkstoff aufweisen. Eine Pelotte kann beispielsweise Silikonkautschuk, Polyurethan oder einen ähnlichen Werkstoff aufweisen oder aus diesem gefertigt sein. Der Druckkörper kann einen Anpressdruck gewährleisten. Der Druckkörper kann eine Formstabilität gewährleisten, wobei der Druckkörper zum einen elastisch, aber auch zum anderen inkompressibel sein kann. Im Sinne der Erfindung erfasst der Begriff Druckkörper einen Körper, der permanent einen Raum ausfüllt. Ein erfindungsgemäßer Druckkörper weist vorzugsweise einen insbesondere festen Körper, der als Festkörper ausgestaltet sein kann, auf, der eine räumliche Ausdehnung besitzt, die mindestens 60 %, bevorzugt mindestens 80 %, des Volumens des Druckkörpers umfasst, die vorzugsweise nur durch Ausübung von Druck auf den Körper veränderbar ist. Der Begriff Druckkörper im Sinne der Erfindung umfasst insbesondere Körper mit einer geschlossenen Hülle, deren eingeschlossenes Volumen unveränderbar ist.

Die Elektrodenanordnung bzw. Teile davon kann bzw. können auf dem Druckkörper selbst ausgestaltet sein. Es können leitfähige Strukturen zur Ausbildung der Elektrodenanordnung bzw. Teilen davon auf der Oberfläche des Druckkörpers vorgesehen sein. In einer Ausführungsform ist die Elektrodenanordnung auf einem Textil, Gewirke oder Stoff ausgebildet. Es kann vorgesehen sein, dass die Elektrodenanordnung ein leitfähiges Textil, das in einem nicht leitfähigen Textil bzw. einem anderen Material eingebettet ist, umfasst. Es kann vorgesehen sein, dass die Elektrodenanordnung leitfähige Fäden umfasst, die in einem nicht leitfähigen Textil oder anderem Material eingewirkt sind. Hierdurch wird eine einfach handhabbare Vorrichtung geschaffen, die einfach herstellbar ist.

Der Begriff Abstandsgewirke erfasst erfindungsgemäß doppelflächige Textilien, bei denen insbesondere die kettengewirkten Warenflächen durch abstandshaltende Verbindungsfäden, sogenannte Polfäden, auf Distanz gehalten werden.

Vorzugsweise können die flächig verteilt angeordneten Elektroden des Elektrodenfelds als Abschnitt einer leitfähigen Struktur auf einem nicht leitfähigen Textil ausgestaltet sein, das mit dem Tragelement unter Ausbildung eines Zwischenraums für den Druckkörper verbunden ist. Das Tragelement und das Textil können eine Tasche für den Druckkörper bilden, in dem der Druckkörper angeordnet ist und keine Befestigung zwischen Druckkörper und Textil bzw. Tragelement vorliegen muss.

Der mit den Elektroden überspannten Fläche kann eine entscheidende Rolle zukommen. So ist es wahrscheinlich, dass eine möglichst große Fläche eines nozizeptiven bzw. rezeptiven Feldes stimuliert werden muss, um ein SEP optimal auszulösen, da auch eine Mindestzahl von Afferenzen gereizt werden sollten, damit das SEP als solches erkannt/gemessen werden kann. Grundsätzlich kann die Größe der mit Elektroden versehenen Fläche des Elektrodenfelds an die entsprechende Größe des rezeptiven Feldes der zu untersuchenden Körperregion leicht verändert werden, was einen technischen Vorteil beispielsweise gegenüber einer konzentrischen Elektrodenanordnung darstellt.

Zur Auslösung eines SEP an Hautafferenzen kann eine erfindungsgemäße Elektrodenanordnung zur Reizbeaufschlagung als Oberflächenelektrode verwendet werden. Durch Aufbringen der Elektrodenanordnung auf der Hautoberfläche wird durch eine elektrische Pulsform, die mittels Frequenz, Pulsbreite und Pulsamplitude (Stromstärke) charakterisiert sein kann, zwischen Anode und Kathode ein elektrisches Feld aufgebaut. Durch dieses elektrische Feld können Afferenzen, die sich in den oberflächlichen Hautschichten befinden, stimuliert werden. Das nozizeptive System besitzt verschiedene Arten Nervenfasern, die mit Hilfe der Elektrostimulation beeinflusst werden können; sogenannte Aδ-Fasern, entsprechen den Faserstrukturen für die Verarbeitung schneller akuter Schmerzen, und C-Fasern vermitteln den dumpfen Schmerz. Die verschiedenen Nervenfasern transportieren dabei die Reizmodalitäten.

Das nozizeptive System hat dabei zwei Arten von Schmerzfasern: Aδ- und C-Nervenfasern. Bei den Aδ-Fasern handelt es sich um relative dicke (ca. 3-5 µm) und aufgrund der Ummantelung durch eine sog. Myelinscheide, schnell leitende (5 - 50 m/s) Fasertypen. Hingegen sind die C-Fasern dünn (ca. 1 µm) und nicht myelinisiert. Sie zeigen Reizleitungsgeschwindigkeiten von unter 1 m/s. Aδ-Fasern enden auf Rückenmarksebene in den Schichten I und V des Hinterhorns, wo sie Synapsen mit Projektionsneuronen bilden. Die meisten C-Fasern hingegen sind mit Interneuronen in Schicht II verschaltet. In den Schichten III und IV des Hinterhorns enden die Axone der Berührungs- und Temperatursensoren.

Bevorzugt sind die Kathoden und/oder Anoden mit einem durch eine Steuereinheit regelbaren Strom von 0,1 bis 30 mA, oder höher, impulsförmig beaufschlagbar. Es kann für den regelbaren Strom bevorzugt für ein Elektrodenfeld mit nicht geschlossener weiterer Elektrode um das Elektrodenfeld herum ein Wert im Bereich von 0,1 mA bis 20 mA, insbesondere bevorzugt 1 mA bis 10 mA, gewählt werden. Es kann für den regelbaren Strom bevorzugt für ein Elektrodenfeld mit um das Elektrodenfeld geschlossener weiterer Elektrode ein Wert im Bereich von 1 mA bis 10mA, insbesondere bevorzugt 0,9 mA bis 4,0 mA, gewählt werden. Bevorzugt können monopolare Rechteckimpulse durch die Steuereinheit erzeugt werden, die einen besonders steilen Anstieg und einen besonders steilen Abfall aufweisen können. Der steile Anstieg und der steile Abfall können derart charakterisiert sein, dass innerhalb von 10 µs die maximale Ausgangsspannung erreicht wird. Die Pulsdauer kann 50 µs bis 2000 µs, insbesondere 100 µs bis 500 µs betragen. Als Pulsfolgefrequenz kann ein Wert zwischen 0,1 Hz bis 10 Hz, insbesondere zwischen 1 und 4 Hz, vorzugsweise insbesondere im Wesentlichen 4Hz, gewählt werden. Es kann auch eine Pulsfolgefrequenz mit einem Wert zwischen 50 Hz bis 100 Hz gewählt werden. Als bevorzugter Wert für die Pulsfolgefrequenz kann ein Wert aus dem Bereich von 0,01 Hz bis 5 Hz, insbesondere 1,5 Hz bis 4,0 Hz, verwendet werden, bei dem gute Small-fiber SEPs gemessen werden können.

Abgesehen von der Amplitude ist die Impulsbreite mitentscheidend für das Faserspektrum, das erregt wird. Je kürzer der Impuls, desto höher muss die Amplitude sein und umgekehrt. Um die zur Schmerzlinderung benötigten großkalibrigen Fasern zu erregen, wird bevorzugt eine ausreichende, meist fixierte Impulsbreite, üblicherweise ungefähr 0,2 bis 0,3 ms, verwendet, um die Voraussetzung zu schaffen, dass die Amplitude reduziert werden kann. Als Impulsform wird vorzugsweise ein Rechteck-Impuls verwendet. Es kann auch vorgesehen sein, dass die Impulsform einen sinusförmigen Verlauf aufweist.

Insbesondere kann vorgesehen sein, dass die Regelung des Stromes zur Beaufschlagung der Elektroden auf einen Konstantwert unter Berücksichtigung der Impulsbreite erfolgt. Hierdurch kann eine bestimmte Energie appliziert werden.

Es hat sich gezeigt, dass mit einer herkömmlichen konzentrischen Elektrode kein Small-fiber SEP in real-time (Echtzeit) aufgenommen werden kann. Die Verwendung eines Elektrodenfelds mit weiterer Elektrode führt zu der Möglichkeit der real-time Erfassung eines SEP. Bei einer flächig um das Elektrodenfeld zumindest teilweise geschlossenen weiteren Elektrode ist eine weitere Verbesserung möglich. Nachfolgend wird die Erfindung an Hand von Zeichnungen näher erläutert, die allerdings lediglich mögliche Ausführungsformen der Erfindung zeigen. Darin zeigen:
- Fig. 1:: eine schematische Darstellung einer Elektrodenanordnung zur Reizbeaufschlagung in einer Draufsicht;
- Fig. 2a:: eine schematische Darstellung einer weiteren Elektrodenanordnung zur Reizbeaufschlagung in einer Draufsicht;
- Fig.2b:: die Elektrodenanordnung gemäß Fig. 2a zur Reizbeaufschlagung in einer isometrischen Darstellung;
- Fig. 3:: zeigt eine Ableitung eines elektrischen Potentials für eine Elektrodenanordnung mit konzentrisch zueinander angeordneten Elektroden für die Reizbeaufschlagung;
- Fig. 4:: zeigt eine Ableitung eines elektrischen Potentials für eine Elektrodenanordnung mit Elektroden in einem Elektrodenfeld und einer beabstandeten weiteren Elektrode; und
- Fig. 5:: zeigt eine Ableitung eines elektrischen Potentials für eine Elektrodenanordnung mit Elektroden in einem Elektrodenfeld und einer beabstandeten weiteren Elektrode, die die Elektroden des Elektrodenfelds geschlossen umgibt.

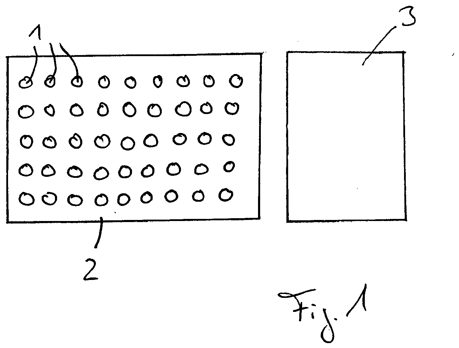

In Fig. 1 ist schematisch eine Elektrodenanordnung zur Reizbeaufschlagung auf der Haut dargestellt. Es ist eine Elektrodenanordnung mit Elektroden 1 dargestellt, die in Anlage mit der Haut eines Menschen gelangen kann. Die Elektroden 1 sind auf oder in einer Trägerstruktur 2 angeordnet, die vorzugsweise allergenfrei ausgestaltet ist. Die Elektroden 1 sind in Form eines Elektrodenfeldes angeordnet. Die Elektroden 1 liegen in einem rechteckförmigen Feld vor, wobei der Abstand zwischen zwei Elektroden 1 jeweils gleich ist. Zudem weist die Elektrodenanordnung eine flächige weitere Elektrode 3 auf, die den Gegenpol zu den Elektroden 1, die mit gleicher Polarität beaufschlagt werden, bildet. Die Elektroden 1 sind aufgrund ihrer entsprechenden Verschaltung miteinander mit identischer Polarität beaufschlagbar.

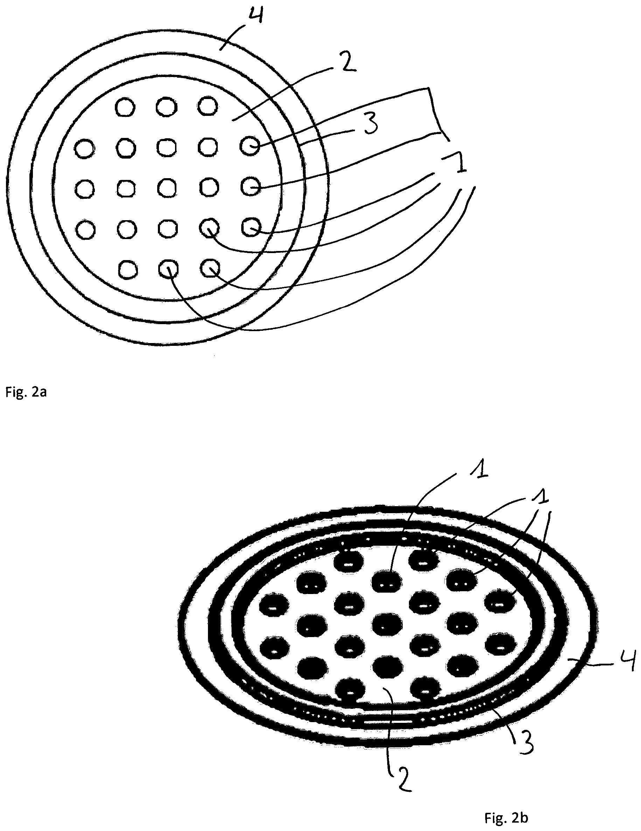

In Fig. 2a, b ist schematisch eine Elektrodenanordnung zur Reizbeaufschlagung auf der Haut dargestellt. Elektroden 1 sind auf einer Trägerstruktur 2 für einen punktuellen Kontakt mit der Haut ausgestaltet. Die Elektroden 1 sind in Form eines Elektrodenfeldes angeordnet. Die Elektroden 1 werden von einer weiteren Elektrode 4 umgeben. Die weitere Elektrode 3 ist geschlossen um die Elektroden 1 des Elektrodenfeldes ausgebildet. Die weitere Elektrode 3 ist als Vorsprung gegenüber einer nicht leitenden Fläche 4 ausgestaltet. Die weitere Elektrode 3 ist kreisförmig. Die Elektroden 1 sind aufgrund ihrer entsprechenden Verschaltung miteinander mit identischer Polarität beaufschlagbar.

Die Elektroden 1 des Elektrodenfelds weisen einen Abstand von etwa 4,5 mm zueinander auf. Die Elektroden 1 des Elektrodenfelds sind auf Gitterpunkten eines Gitters mit 4,5 mm Abstand angeordnet. Die Elektroden 1 des Elektrodenfelds weisen einen Radius von 0,5 mm auf. Es liegt eine Punktsymmetrie der Elektroden 1 des Elektrodenfelds in Bezug auf den Mittelpunkt des Elektrodenfelds vor. Der Mittelpunkt des Elektrodenfelds wird durch die in Zeile 3 und Spalte 3 angeordnete Elektrode 1 gebildet. Der Abstand der weiteren Elektrode zum Mittelpunkt des Elektrodenfeldes beträgt 30 mm.

Zum Betreiben der Elektrodenanordnung zur Reizbeaufschlagung bzw. der elektrischen Beaufschlagung der Elektroden ist ein bekanntes Standard-SEP-Gerät vorgesehen, das mit den Elektroden elektrisch, beispielsweise in Form einer Steckverbindung, verbunden werden kann. Die Steuereinheit innerhalb des Standard-SEP-Geräts kann die Elektroden der Elektrodenanordnung mit Pulsen beaufschlagen.

Zum Ableiten des SEP ist ebenfalls das bekannte Standard-SEP-Gerät vorgesehen, das auch mit den Ableitelektroden elektrisch beispielsweise in Form einer Steckverbindung verbunden werden kann. Die Messeinheit innerhalb des Standard-SEP-Geräts kann das an den Ableitelektroden anliegende Signal aufnehmen und/oder darstellen.

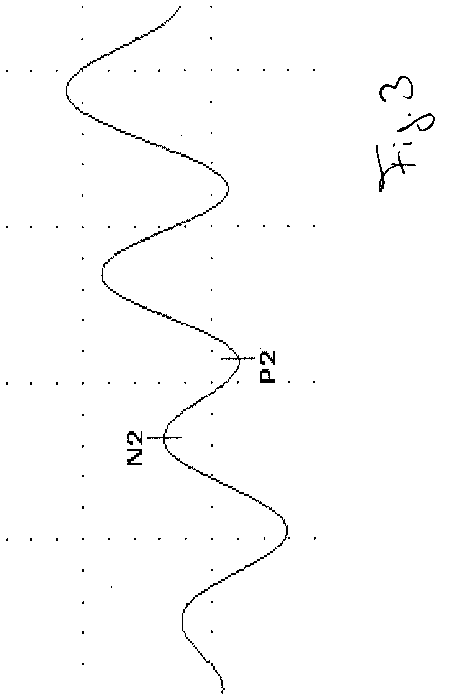

Die Fig. 3 bis 5 zeigen jeweils ein in Echtzeit abgeleitetes elektrisches Potential.

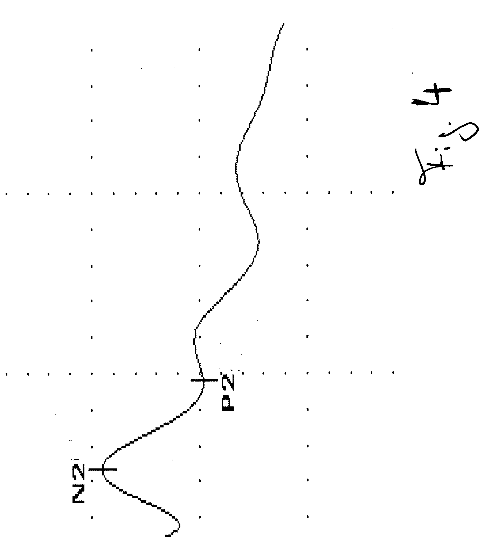

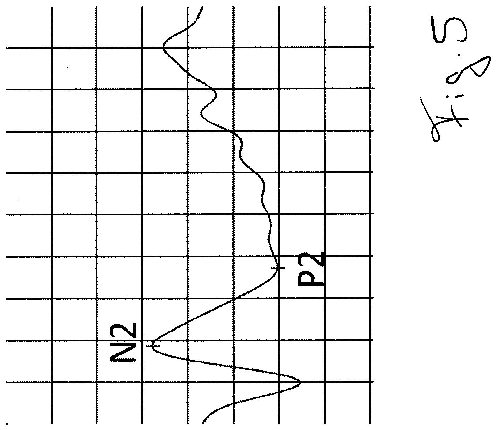

Die Fig. 3 zeigt eine Ableitung eines elektrischen Potentials für eine Elektrodenanordnung mit konzentrisch zueinander angeordneten Elektroden für die Reizbeaufschlagung, wie diese aus dem Stand der Technik bekannt ist. Es ergibt sich kein klares Signal. Stattdessen liegt die Vermutung nahe, dass ein 50 Hz-Rauschen (Versorgungspannung) vorliegt. Lediglich zum Zwecke des Vergleiches mit den in Fig. 4 und Fig. 5 dargestellten Potentialen sind ein N2 und ein P2 markiert.

Die Fig. 4 zeigt eine Ableitung eines elektrischen Potentials für eine Elektrodenanordnung mit Elektroden in einem Elektrodenfeld und einer beabstandeten weiteren Elektrode. Die Elektroden des Elektrodenfelds sind in 9 Spalten und 5 Zeilen angeordnet. Die weitere Elektrode ist eine zum Elektrodenfeld beabstandete, insbesondere flächige, Elektrode. Es ist ein klares Signal erkennbar.

Die Fig. 5 zeigt eine Ableitung eines elektrischen Potentials für eine Elektrodenanordnung mit Elektroden in einem Elektrodenfeld und einer beabstandeten weiteren Elektrode, die die Elektroden des Elektrodenfelds geschlossen umgibt (gemäß Fig. 2). Es ist ein klares Signal erkennbar, welches im Vergleich zu dem in Fig. 4 dargestellten Potential eine höhere Amplitude aufweist.

## Patentansprüche

1. Vorrichtung zur Diagnose der Funktionalität von Nervenfasern, aufweisend eine Elektrodenanordnung zur Reizbeaufschlagung, die mehrere in einem Elektrodenfeld angeordnete Elektroden (1) und mindestens eine weitere Elektrode (3) umfasst, und mindestens eine Ableitelektrode, wobei die Vorrichtung ferner eine Steuereinheit zur Beaufschlagung der Elektroden (1) des Elektrodenfelds mit Pulsen und eine mit der Ableitelektrode verbundene Messeinheit zum Erfassen eines somatosensibel evozierten Potentials (SEP) dünner Nervenfasern aufweist, wobei die weitere Elektrode (3) zum Elektrodenfeld beabstandet ist, und die Elektroden (1) des Elektrodenfelds flächig verteilte punktuell ausgebildete Elektroden sind, die eine verjüngende Oberfläche zur punktuellen Kontaktierung der Haut aufweisen und ausgestaltet sind, auf die Haut ohne Perforation derselben aufgelegt zu werden.

2. Vorrichtung nach Anspruch 1, **dadurch gekennzeichnet, dass** die Steuereinheit ausgestaltet ist, die Elektroden (1) mit einem regelbaren Strom von 0,1 bis 30 mA impulsförmig zu beaufschlagen.

3. Vorrichtung nach Anspruch 1 oder 2, wobei die Steuereinheit ausgestaltet ist, die Elektroden (1) des Elektrodenfelds mit einer anderen Polarität als der Polarität der weiteren Elektrode (3) zu beaufschlagen.

4. Vorrichtung nach Anspruch 1 bis 3, wobei die weitere Elektrode (3) flächig ausgestaltet ist.

5. Vorrichtung nach einem der Ansprüche 1 bis 4, wobei die weitere Elektrode (3) einen gegenüber einer Fläche (4) ausgebildeten Vorsprung aufweist.

6. Vorrichtung nach einem der Ansprüche 1 bis 5, wobei die mindestens eine Ableitelektrode als Nadelelektrode ausgestaltet ist.

7. Vorrichtung nach einem der Ansprüche 1 bis 6, wobei die Elektroden (1) des Elektrodenfelds in Zeilen und Spalten angeordnet sind, und die Anzahl der Zeilen 7 bis 11 und die Anzahl der Spalten 3 bis 7 ist und der Abstand der Zeilen im Bereich von 2,0 mm bis 10 mm und der Abstand der Spalten im Bereich von 2,0 mm bis 10 mm liegt.

8. Vorrichtung nach einem der Ansprüche 1 bis 7, wobei die weitere Elektrode (3) die Elektroden (1) des Elektrodenfelds zumindest teilweise umgibt.

9. Vorrichtung nach Anspruch 8, wobei die weitere Elektrode (3) die Elektroden (1) des Elektrodenfelds geschlossen umgibt.

10. Vorrichtung nach einem der Ansprüche 1 bis 9, wobei ein Abstand zwischen Elektroden (1) des Elektrodenfelds, die zu der weiteren Elektrode (3) benachbart sind, und der weiteren Elektrode (1) im Bereich von 0,5 mm bis 6,0 mm liegt.

11. Vorrichtung nach einem der Ansprüche 1 bis 10, wobei die Elektroden (1) des Elektrodenfelds gleichmäßig im Elektrodenfeld angeordnet sind.

12. Vorrichtung nach einem der Ansprüche 1 bis 11, wobei eine kürzeste Verbindungslinie entlang benachbarter äußerer Elektroden (1) des Elektrodenfelds eine symmetrische Fläche bildet.

13. Vorrichtung nach einem der Ansprüche 1 bis 12, wobei der Abstand zwischen den äußeren Elektroden (1) des Elektrodenfelds und der weiteren Elektrode (3) für die äußeren Elektroden (1) variiert.

14. Vorrichtung nach einem der Ansprüche 1 bis 13, wobei der Abstand zwischen den äußeren Elektroden (1) des Elektrodenfelds und der weiteren Elektrode (3) für die äußeren Elektroden (1) im Wesentlichen gleich ist.

15. Vorrichtung nach einem der Ansprüche 1 bis 14, wobei das Verhältnis der Gesamtgröße der Elektroden (1) des Elektrodenfelds zu der Größe der weiteren Elektrode (3) kleiner als 0,7, insbesondere kleiner als 0,5, ist.

## Claims

1. Device for diagnosing the functionality of nerve fibres, having an electrode arrangement for stimulus charging, comprising several electrodes (1) arranged in an electrode array and at least one further electrode (3), and at least one recording electrode, wherein the device also has a control unit for stimulus charging of the electrodes (1) of the electrode array with pulses and a measuring unit connected to the recording electrode for detecting a somatosensory evoked potential (SEP) of thin nerve fibres, wherein the further electrode (3) is distanced from the electrode array, and the electrodes (1) of the electrode array are two-dimensionally distributed punctiform electrodes which have a tapering surface for punctiform contact with the skin and are designed to be laid on the skin without perforating same.

2. Device according to claim 1, **characterised in that** the control unit is designed to charge the electrodes (1) with an adjustable, pulse-shaped current of 0.1 to 30 mA.

3. Device according to claim 1 or 2, wherein the control unit is designed to charge the electrodes (1) of the electrode array with a polarity which is different from the polarity of the further electrode (3).

4. Device according to claim 1 to 3, wherein the further electrode (3) is designed to be two-dimensional.

5. Device according to one of claims 1 to 4, wherein the further electrode (3) has a projection which is designed to be opposite a surface (4).

6. Device according to one of claims 1 to 5, wherein the at least one recording electrode is designed to be a needle electrode.

7. Device according to one of claims 1 to 6, wherein the electrodes (1) of the electrode array are arranged in lines and columns, and the number of lines is 7 to 11 and the number of columns 3 to 7, and the space between lines is in the range of 2.0 mm to 10 mm and the space between columns is in the range of 2.0 mm to 10 mm.

8. Device according to one of claims 1 to 7, wherein the further electrode (3) at least partially surrounds the electrodes (1) of the electrode array.

9. Device according to claim 8, wherein the further electrode (3) surrounds the electrodes (1) of the electrode array in closed manner.

10. Device according to one of claims 1 to 9, wherein a distance between electrodes (1) of the electrode array adjacent to the further electrode (3) and the further electrode (1) is in the region of 0.5 mm to 6.0 mm.

11. Device according to one of claims 1 to 10, wherein the electrodes (1) of the electrode array are arranged uniformly in the electrode array.

12. Device according to one of claims 1 to 11, wherein a shortest connection line along adjacent outer electrodes (1) of the electrode array forms a symmetrical surface.

13. Device according to one of claims 1 to 12, wherein the distance between the outer electrodes (1) of the electrode array and the further electrode (3) is varied for the outer electrodes (1).

14. Device according to one of claims 1 to 13, wherein the distance between the outer electrodes (1) of the electrode array and the further electrode (3) for the outer electrodes (1) is substantially the same.

15. Device according to one of claims 1 to 14, wherein the ratio of the total size of the electrodes (1) of the electrode array to the size of the further electrode (3) is less than 0.7, in particular less than 0.5.

## Revendications

1. Dispositif de diagnostic de la fonctionnalité des fibres nerveuses, présentant un ensemble d'électrodes pour l'application d'un stimulus, qui comprend plusieurs électrodes (1) disposées dans un champ d'électrodes et au moins une autre électrode (3), et au moins une électrode de dérivation, le dispositif présentant en outre une unité de commande pour l'application d'impulsions aux électrodes (1) du champ d'électrodes et une unité de mesure reliée à l'électrode de dérivation pour la détection d'un potentiel évoqué somatosensible (SEP) de fibres nerveuses minces, l'autre électrode (3) étant espacée du champ d'électrodes et les électrodes (1) du champ d'électrodes étant des électrodes réparties en surface, formées par points, qui présentent une surface effilée pour le contact ponctuel avec la peau et qui sont conçues pour être appliquées sur la peau sans perforer celle-ci.

2. Dispositif selon la revendication 1, **caractérisé en ce que** l'unité de commande est conçue pour alimenter par impulsions les électrodes (1) avec un courant réglable de 0,1 à 30 mA.

3. Dispositif selon la revendication 1 ou 2, dans lequel l'unité de commande est conçue pour appliquer aux électrodes (1) du champ d'électrodes une polarité différente de la polarité de l'autre électrode (3).

4. Dispositif selon les revendications 1 à 3, dans lequel l'autre électrode (3) est de forme plate.

5. Dispositif selon l'une quelconque des revendications 1 à 4, dans lequel l'autre électrode (3) présente une saillie formée par rapport à une surface (4).

6. Dispositif selon l'une quelconque des revendications 1 à 5, dans lequel l'au moins une électrode de dérivation est conçue comme une électrode à aiguille.

7. Dispositif selon l'une quelconque des revendications 1 à 6, dans lequel les électrodes (1) du champ d'électrodes sont disposées en lignes et en colonnes, et le nombre de lignes est de 7 à 11 et le nombre de colonnes est de 3 à 7, et l'espacement des lignes est compris dans la plage de 2,0 mm à 10 mm et l'espacement des colonnes est compris dans la plage de 2,0 mm à 10 mm.

8. Dispositif selon l'une quelconque des revendications 1 à 7, dans lequel l'autre électrode (3) entoure au moins partiellement les électrodes (1) du champ d'électrodes.

9. Dispositif selon la revendication 8, dans lequel l'autre électrode (3) entoure de manière fermée les électrodes (1) du champ d'électrodes.

10. Dispositif selon l'une quelconque des revendications 1 à 9, dans lequel une distance entre des électrodes (1) du champ d'électrodes, qui sont adjacentes à l'autre électrode (3), et l'autre électrode (1) est comprise dans la plage de 0,5 mm à 6,0 mm.

11. Dispositif selon l'une quelconque des revendications 1 à 10, dans lequel les électrodes (1) du champ d'électrodes sont disposées uniformément dans le champ d'électrodes.

12. Dispositif selon l'une quelconque des revendications 1 à 11, dans lequel une ligne de connexion la plus courte le long d'électrodes extérieures adjacentes (1) du champ d'électrodes forme une surface symétrique.

13. Dispositif selon l'une quelconque des revendications 1 à 12, dans lequel la distance entre les électrodes extérieures (1) du champ d'électrodes et l'autre électrode (3) varie pour les électrodes extérieures (1).

14. Dispositif selon l'une quelconque des revendications 1 à 13, dans lequel la distance entre les électrodes extérieures (1) du champ d'électrodes et l'autre électrode (3) est essentiellement la même pour les électrodes extérieures (1).

15. Dispositif selon l'une quelconque des revendications 1 à 14, dans lequel le rapport entre la taille totale des électrodes (1) du champ d'électrodes et la taille de l'autre électrode (3) est inférieur à 0,7, notamment inférieur à 0,5.
